# EUROPÄISCHE PATENTSCHRIFT

(11) **EP 2 845 616 B1**
(45) Veröffentlichungstag und Bekanntmachung des Hinweises auf die Patenterteilung: **09.01.2019**
(21) Anmeldenummer: 13183413.7
(22) Anmeldetag: 06.09.2013
(51) Int. Cl.: A61B 5/0488, A61B 5/08, A61M 16/00, A61B 5/00

(54) **VORRICHTUNG ZUM ERZEUGEN EINES STEUERSIGNALS FÜR EIN BEATMUNGSGERÄT**
DEVICE FOR GENERATING A CONTROL SIGNAL FOR A RESPIRATORY APPARATUS
DISPOSITIF DE GÉNÉRATION D'UN SIGNAL DE COMMANDE POUR UN APPAREIL RESPIRATOIRE

(43) Veröffentlichungstag der Anmeldung: 11.03.2015
(73) Patentinhaber: Drägerwerk AG & Co. KGaA, 23558 Lübeck (DE)
(72) Erfinder: Kahl, Lorenz, 23558 Lübeck (DE); Eger, Marcus, 23558 Lübeck (DE)

(56) Entgegenhaltungen:
- WO-A1-01/19440
- WO-A1-02/094358
- DE-A1-102010 055 242
- DE-A1-102010 055 243
- DE-B3-102007 062 214
- DE-T2- 69 921 782

## Beschreibung

Die vorliegende Erfindung betrifft eine Vorrichtung zum Erzeugen eines Steuersignals für ein Beatmungsgerät.

Mit einem mit einer solchen Vorrichtung durchgeführten Verfahren kann ein Beatmungsgerät, mit dessen Hilfe ein Patient künstlich beatmet wird, angesteuert werden, wobei aus einem Messsignal, das am Patienten gewonnen wird, ein Steuersignal erzeugt wird, das das Beatmungsgerät zwischen einer Inspirationsphase, in der dem Patienten Atemluft zugeführt wird, und einer Exspirationsphase, bei der der Patient ausatmet, hin und herschaltet. Aus dem Messsignal wird dabei bestimmt, wann der Patient von sich aus, wenn auch unzureichend, Anstrengungen unternimmt, einzuatmen bzw. dies unterlässt, um auszuatmen.

Aus dem Stand der Technik ist es dazu bereits bekannt, Strömungssensoren zu verwenden, die den Volumenstrom in einem Schlauchabschnitt zwischen dem Beatmungsgerät und dem Patienten erfassen, und je nach Richtung und Größe dieses Volumenstroms wird das Beatmungsgerät dann in die Inspirations- bzw. die Exspirationsphase geschaltet.

Eine derartige Vorgehensweise ist jedoch nachteilhaft, weil es für ein Umschalten erforderlich ist, dass der Patient eine vergleichsweise hohe Anstrengung unternommen haben muss, um eine messbare Änderung des Volumenstroms hervorzurufen, die dann dazu führt, dass das Beatmungsgerät umgeschaltet wird. Ein weiterer Nachteil ist, dass die Umschaltung erst dann erfolgt, wenn der Patient bereits Anstrengungen unternommen und die Atmung nicht schon zu einem früheren Zeitpunkt unterstützt.

Vor diesem Hintergrund ist es wünschenswert, statt des Volumenstromsignals ein anderes physiologisches Signal zur Steuerung des Beatmungsgeräts zu verwenden, das deutlich sensitiver auf den Beginn der Atemanstrengungen ist als ein Volumenstromsignal.

Hierzu ist es aus dem Stand der Technik wie beispielsweise der EP 1 056 499 B1 oder U.S. 4,915,103 bereits bekannt, das Elektromyogramm-Signal zu verwenden, wobei hier jedoch das Problem auftritt, dass das mit Elektroden auf der Körperoberfläche oder, wie in der EP 1 056 499 B1 offenbart, mit einer Elektrode in der Luftröhre erfasste Signal, einen erheblichen Anteil an Störungen aufweist. Außerdem sind die Potentiale, die dabei erfasst werden und mit denen atmungsrelevante Muskeln angeregt werden, sehr klein. Insbesondere im Vergleich zu den Herzsignalen (EKG-Signale) sind diese Signale um Größenordnungen kleiner, sodass die Herzsignale maßgebliche Artefakte darstellen.

Daher bedarf es großer Anstrengungen, um in dem Elektromyogramm-Signal den Übergang zwischen einer Phase, bei der keine Anstrengungen durch den Patienten vorliegen und die die Exspirationsphase wiederspiegelt, und einer Inspirationsphase, bei der die für die Atmung relevanten Muskeln angeregt werden, zu detektieren. Allerdings bietet das Elektromyogramm-Signal, das sensitiv ist auf eine Änderung der Anregungspotentiale für die atmungsrelevanten Muskeln, den großen Vorteil, dass mit diesem Signal der Beginn der Atemanstrengungen sehr gut erfasst werden kann.

DE 10 2010 055 242 A1 beschreibt ein Verfahren zum Steuern eines Beatmungsgerätes, nämlich zum aufeinanderfolgenden Umschalten zwischen Inspiration und Exspiration, indem ein aufgenommenes Atemaktivitätssignal auf ein Schwellenkriterium für die Umschaltung in die nächste Beatmungsphase hin untersucht und bei erfülltem Schwellenkriterium in die nächste Beatmungsphase umgeschaltet wird. Entscheidend für das Verfahren nach DE 10 2010 055 242 A1 ist, dass die von dem Beatmungsgerät gesteuerte Beatmungsgröße beim Umschalten nicht in einem Schritt vom Istwert auf den Zielwert für die neue Beatmungsphase umgeschaltet wird, sondern der Übergang bei der Umschaltung der Beatmungsphase in viele Teilschritte unterteilt wird. Bei der Umschaltung der Beatmungsphase wird dann nach jedem Teilschritt das aktuelle Atemaktivitätssignal wieder auf das für die Auslösung der gegenwärtigen Umschaltung verantwortliche Schwellenkriterium hin erneut untersucht und, falls dieses Schwellenkriterium nach einem Teilschritt nicht mehr erfüllt ist, automatisch in den Betriebszustand des Beatmungsgerätes vor der letzten Umschaltung zurückgesprungen wird, und anderenfalls mit dem nächsten Teilschritt der Änderung der Beatmungsgröße vom Istwert zum Zielwert fortgefahren wird. Bei einer kurzzeitigen Störung kann so zwar der Übergang in die nächste Beatmungsphase eingeleitet werden, der Fehler wird aber bemerkt und der Übergang abgebrochen, sobald das auslösende Schwellenkriterium nicht mehr erfüllt ist.

WO 01/19440 A1 beschreibt ein Verfahren zur unterstützenden Beatmung, das den Beatmungsdruck bezüglich Amplitude und Phase angepasst an dem Atemzyklus des Patienten bestimmt, wobei sowohl der respiratorische Volumenstrom als auch die Atemanstrengung des Patienten eingehen, wobei letztere durch ein Elektromyogramm-Signal abgeleitet sein kann. Die Eingangsgrößen respiratorischer Volumenstrom und Atemanstrengungssignal werden mit einer Fuzzy-Logik nach vorgegebenen Regeln verarbeitet, um den zu liefernden Beatmungsunterstützungsdruck zu bestimmen, der dann von dem Beatmungsgerät geliefert wird.

DE 10 2010 055 243 A1 beschreibt ein Verfahren zur Steuerung eines Beatmungsgeräts, um aufeinanderfolgend zwischen Inspiration und Exspiration umzuschalten, wozu z.B. ein Elektromyogramm-Signal als Atemanstrengungssignal verwendet wird. Das EMG-Signal wird dabei mit dynamischen Schwellen verglichen. Das bedeutet, dass in einer Exspirationsphase ein dynamischer Schwellenwertverlauf für die Umschaltung in die Inspirationsphase verwendet wird, der nach Beginn der gegenwärtigen Exspirationsphase bis zu dem Ende einer ausgewählten inspiratorischen Refraktärzeit auf hohen Werten gehalten wird, um ein Umschalten in die Inspiration zu verhindern, und danach auf einen inspiratorischen Schwellenwert abgesenkt wird. In der Inspirationsphase wird die dynamische Schwelle umgekehrt nach Beginn der gegenwärtigen Inspirationsphase zunächst auf niedrigen Werten gehalten und danach auf einen exspiratorischen Schwellzielwert angehoben. Um den Verlauf der dynamischen Schwellen als Funktion der Zeit festzulegen, kann vorgesehen sein, dass zu bestimmten Zeitpunkten der Atmungsphasen Häufigkeitsverteilungen der EMG-Signale erfasst werden. Dann kann der dynamische Schwellenverlauf als Funktion der Zeit so festgelegt werden, dass die Wahrscheinlichkeit der Umschaltung der Beatmungsphase aufgrund der aufgenommenen Häufigkeitsverteilungen den Verteilungen der Dauern der Inspirations- und Exspirationsphasen folgt.

Der Stand der Technik liefert jedoch keine sehr zuverlässigen Verfahren, mit denen in einem Elektromyogramm-Signal, aufgenommen im Bereich der atmungsrelevanten Muskeln, eine Unterscheidung zwischen der Inspirationsphase und der Exspirationsphase gemacht werden kann und darauf basierend ein Beatmungsgerät angesteuert werden kann.

Daher ist es ausgehend vom Stand der Technik die Aufgabe der vorliegenden Erfindung, eine Vorrichtung zum Erzeugen eines Steuersignals für ein Beatmungsgerät auf der Grundlage eines Elektromyogramm-Signals bereitzustellen, das den Übergang zwischen Inspirations- und Exspirationsphase zuverlässig bestimmt.

Hierin wird ein Verfahren mit folgenden Schritten beschrieben:
- Erfassen eines Elektromyogramm-Signals (E) das zeitlich aufeinanderfolgende Signalwerte (x) umfasst,
- Transformieren des Elektromyogramm-Signals (E) in ein Bewertungssignal (F, G) durch Anwenden einer Bewertungsfunktion auf das Elektromyogramm-Signal (E), wobei beim Transformieren einem Signalwert (x) des Elektromyogramm-Signals (E) ein Signalwert (y) des Bewertungssignals (F, G) zugewiesen wird, wobei die Bewertungsfunktion durch einen Hauptparametersatz bestimmt ist, sodass der Hauptparametersatz festlegt, welchem Signalwert (x) des Elektromyogramm-Signals (E) beim Transformieren bei Anwendung der Bewertungsfunktion welcher Signalwert (y) des Bewertungssignals (F, G) zugewiesen wird, und wobei die Höhe des Signalwerts (y) des Bewertungssignals (F, G) angibt, ob der Signalwert (x) des Elektromyogramm-Signals (E) der Inspirationsphase oder der Exspirationsphase zuzuordnen ist, wobei bei Anwendung der Bewertungsfunktion auf Signalwerte (x) aus einem Intervall um einen Basiswert ein Signalwert (y) des Bewertungssignals (F, G) zugewiesen wird und die Bewertungsfunktion der Logarithmus des Verhältnisses der Wahrscheinlichkeiten P_{I}(x), P_{E}(x) ist, dass der Signalwert (x) des Elektromyogramm-Signals (E) zur Inspirationsphase oder zur Exspirationsphase gehört,
- Generieren eines Steuersignals (H) aus zeitlich aufeinanderfolgenden Signalwerten, das angepasst ist, ein Beatmungsgerät in eine Inspirationsbetriebsart zu schalten, wenn das Bewertungssignal (F, G) dem Inspirationszustand entspricht, und in eine Exspirationsbetriebsart zu schalten, wenn das Bewertungssignal (F, G) dem Exspirationszustand entspricht,
   wobei parallel zum Transformieren des Elektromyogramm-Signals (E) und dem Generieren des Steuersignals (H) folgende Schritte durchgeführt werden:
   - Transformieren eines Analyseabschnitts (E') des Elektromyogramm-Signals (E) in ein zweites Bewertungssignal (F', G') durch Anwenden der Bewertungsfunktion auf den Analyseabschnitt (E'), wobei beim Transformieren einem Signalwert (X) des Analyseabschnitts (E') ein Signalwert (Y) des zweiten Bewertungssignals (F', G') zugewiesen wird, wobei die Bewertungsfunktion durch einen Analyseparametersatz bestimmt ist, sodass der Analyseparametersatz festlegt, welchem Signalwert (X) des Analyseabschnitts (E') beim Transformieren bei Anwendung der Bewertungsfunktion welcher Signalwert (Y) des zweiten Bewertungssignals (F', G') zugewiesen wird, und wobei die Höhe des Signalwerts (Y') des zweiten Bewertungssignals (F', G') angibt, ob der korrespondierende Signalwert (X) des Analyseabschnitts (E') einer Inspirationsphase oder einer Exspirationsphase entspricht,
   - Bestimmen einer ersten Häufigkeitsverteilung H₁(X) für die Signalwerte (X) aus den Teilen des Analyseabschnitts (E'), für die das zweite Bewertungssignal (F', G') angibt, dass die Inspirationsphase vorliegt,
   - Bestimmen einer zweiten Häufigkeitsverteilung H₂(X) für die Signalwerte (X) aus den Teilen des Analyseabschnitts (E'), für die das zweite Bewertungssignal (F', G') angibt, dass die Exspirationsphase vorliegt,
   - Bestimmen eines erneuerten Parametersatzes aus den Häufigkeitsverteilungen H₁(X), H₂(X) und
   - Übergeben des erneuerten Parametersatzes als Hauptparametersatz.
Analog weist die erfindungsgemäße Vorrichtung neben einem Signaleingang zum Erfassen des Elektromyogramm-Signals die entsprechenden Mittel auf, um die zuvor genannten Verfahrensschritte auszuführen.

Bei dem hierin beschriebenen Verfahren bzw. mit der erfindungsgemäßen Vorrichtung wird für jeden Signalwert des Elektromyogramm-Signals ein Signalwert eines Bewertungssignals durch Anwenden einer Bewertungsfunktion bestimmt, die wiederum durch einen Hauptparametersatz festgelegt ist, sodass der Signalwert des Bewertungssignals eindeutig über den Hauptparametersatz definiert ist. Der Signalwert des Bewertungssignals ist ein Maß dafür, ob der fragliche Signalwert der Inspirationsphase oder der Exspirationsphase zuzuordnen ist.

Die Bewertungsfunktion ist dabei eine Funktion des Verhältnisses der Wahrscheinlichkeiten P_{I}(x), P_{E}(x), dass der fragliche Signalwert x zu der Inspirationsphase und zu der Exspirationsphase gehört, wobei der Logarithmus des Quotienten dieser Wahrscheinlichkeiten, lg(P_{I}(x)/P_{E}(x)), als Bewertungsfunktion verwendet wird. Die Werte für P_{I}(x) und P_{E}(x) bzw. der Quotient aus dem Logarithmus bildet zumindest teilweise den Hauptparametersatz, und die Bewertungsfunktion weist den Signalwerten x den entsprechenden Wert für den Logarithmus aus dem Quotienten der Wahrscheinlichkeiten P_{I}(x), P_{E}(x) zu.

Ist die Wahrscheinlichkeit P_{I}(x) für die Inspirationsphase größer als die für die Exspirationsphase P_{E}(x), ist der Quotient größer 1, sodass der Logarithmus größer 0 ist. Umgekehrt ist die Bewertungsfunktion kleiner 0, wenn die Wahrscheinlichkeit P_{E}(x) dafür, dass der Wert x zu der Exspirationsphase gehört, größer ist als die Wahrscheinlichkeit P_{I}(x) dafür, dass er in der Inspirationsphase erfasst wird, da dann der Quotient (P_{I}(x)/P_{E}(x)) kleiner 1 ist. Daher kann durch einen anschließenden Schwellwertvergleich mit dem Schwellwert Null in einfacher Weise bestimmt werden, ob die Inspirations- oder die Exspirationsphase vorliegt.

Die Transformation unter Verwendung der genannten Bewertungsfunktion schafft insgesamt die Möglichkeit, dass die erfassten Signalwerte danach bewertet werden, zu welcher der beiden Phasen sie gehören. Der Parametersatz kann dabei an den jeweiligen Patienten und die Bedingungen, unter denen das Elektromyogramm-Signal erfasst wird, angepasst sein. Insbesondere wird die Möglichkeit geschaffen, auch während des laufenden Verfahrens den Parametersatz an sich ändernde Bedingungen anzupassen. In jedem Fall wird durch den variablen Parametersatz aber erreicht, dass das Verfahren ein Steuersignal nicht beispielsweise einfach auf der Grundlage eines Vergleichs mit einem fest vorgegebenen Schwellwerts generiert, sondern flexibel arbeiten kann.

Ferner ist es bevorzugt, wenn entweder das Elektromyogramm-Signal oder das durch Transformieren erzeugte Bewertungssignal geglättet werden. Dies kann in der Weise erfolgen, dass jeder Signalwert des Elektromyogramm-Signals oder des Bewertungssignals als vorgegebener Signalwert behandelt wird und dass jeder vorgegebene Signalwert des Elektromyogramm-Signals oder des Bewertungssignals ersetzt wird durch einen Mittelwert aus Signalwerten des jeweiligen Signals, die den vorgegebenen Signalwert umfassen können und eine erste Anzahl von zeitlich vor dem bestimmten Signalwert liegenden Signalwerten umfassen.

Durch eine solche Glättung werden Spitzen in dem jeweiligen Signal beseitigt. Dabei ist darauf hinzuweisen, dass unter dem Begriff "Mittelwert" nicht nur das arithmetische Mittel verstanden werden soll. Vielmehr wird durch diesen Begriff auch erfasst, dass der Mittelwert in der Weise gebildet werden kann, dass die einzelnen Signalwerte aus der ersten Anzahl unterschiedlich gewichtet in die Bildung des Mittelwerts eingehen. Darüber hinaus sollen unter den Begriff "Mittelwert" im Sinne der vorliegenden Anmeldung auch solche Werte fallen, die durch Anwendung nichtlinearer Glättungsmethoden wie Rangordnungsfilter oder Medianfilter gewonnen werden.

Bei dieser bevorzugten Ausführungsform des hierin beschriebenen Verfahrens wird so vorgegangen, dass die Signalwerte des Elektromyogramm-Signals oder des Bewertungssignals in der Weise bearbeitet werden, dass sie in der Reihenfolge, in der sie erfasst bzw. für die Signalwerte des Bewertungssignals die entsprechenden Elektromyogramm-Signale erfasst wurden, als vorgegebene Signalwerte behandelt werden. Für jeden vorgegebenen Signalwert wird dann aus einer ersten Anzahl von zeitlich vor dem vorgegebenen Signalwert erfassten Signalwerten ein Mittelwert gebildet und der vorgegebene Signalwert durch diesen Mittelwert ersetzt.

Das bedeutet, dass die erste Anzahl von Signalwerten aus Werten besteht, für die die zuvor beschriebene Mittelwertbildung schon durchgeführt wurde. Dies kann mit dem Nachteil verbunden sein, dass bei einer Anwendung dieses Glättungsverfahrens auf das Bewertungssignal ein Anstieg oder ein Abfall des geglätteten Signals dort erst verzögert auftritt. Dies ergibt sich dadurch, dass ein vorgegebener Signalwert nach der Glättung und dem Ersetzen durch den Mittelwert erst dann in seinem Wert ansteigt, wenn die zeitlich vor diesem vorgegebenen Signalwert liegenden Werte auch schon erhöht sind, damit sich dies entsprechend bei dem Mittelwert auswirkt.

Um dieses Problem zumindest teilweise zu beseitigen, kann der Mittelwert in der Weise gebildet werden, dass zeitlich nahe an dem vorgegebenen Signalwert liegende Werte stärker gewichtet in die Mittelwertbildung eingehen als solche aus der ersten Anzahl von Signalwerten, die zeitlich weiter vor dem vorgegebenen Signalwert liegen.

In bevorzugter Weise liegt der Hauptparametersatz als Tabelle vor, d.h. die Vorrichtung ist auch entsprechend ausgestaltet, den Hauptparametersatz als eine Tabelle zu speichern, in der für jeden möglichen Signalwert des Elektromyogramm-Signals der zugewiesene Signalwert für das Bewertungssignal enthalten ist, und wobei bei der Anwendung der Bewertungsfunktion einem Signalwert des Elektromyogramm-Signals der der Tabelle entsprechende Wert als Signalwert des Bewertungssignals zugewiesen wird.

Eine solche Tabelle ermöglicht zum einen eine schnelle Verarbeitung des kontinuierlich erfassten Elektromyogramm-Signals. Zum anderen kann die Tabelle leicht überschrieben werden, wenn sich während des Durchlaufs des Verfahrens bei der Steuerung des Beatmungsgeräts die Bedingungen geändert haben, sodass die Zuordnung der Signalwerte zu der Inspirationsphase und zu der Exspirationsphase eine andere ist.

Ferner ist es bevorzugt, wenn beim Generieren des Steuersignals jeder Signalwert des Bewertungssignals mit einem Schwellwert verglichen wird, wobei das Steuersignal einen ersten Steuerwert annimmt, wenn der Signalwert des Bewertungssignals oberhalb des Schwellwerts liegt, und wobei das Steuersignal einen zweiten Steuerwert annimmt, wenn der Signalwert des Bewertungssignals unterhalb des Schwellwerts liegt.

Auf diese Weise kann das Steuersignal einfach und schnell generiert werden. Der Schwellwert kann dabei an die Bewertungsfunktion angepasst sein. Der Schwellwert ist gleich Null, da als Bewertungsfunktion der Logarithmus aus dem Quotient der Wahrscheinlichkeiten P_{I}(x), P_{E}(x) dafür genommen wird, dass der Signalwert x des Elektromyogramm-Signals zur Inspirationsphase oder zur Exspirationsphase gehört. Trotzdem kann das Verfahren durch Adaptieren des Hauptparametersatzes an geänderte Bedingungen angepasst werden. Diese Möglichkeit führt aber nicht dazu, dass die Generierung des Steuersignals verlangsamt wird.

Wie bereits erläutert, ist vorgesehen, dass bei Anwendung der Bewertungsfunktion auf Signalwerte x aus einem Intervall um einen Basiswert ein Signalwert des Bewertungssignals zugewiesen wird, der eine Funktion des Verhältnisses der Wahrscheinlichkeit P_{I}(x), dass der Signalwert x dem Inspirationszustand entspricht, zu der Wahrscheinlichkeit P_{E}(x), dass der Signalwert x dem Exspirationszustand entspricht, ist. Bei dieser Funktion handelt es sich um den Logarithmus, was dann dazu führt, dass, wie bereits erläutert, der Wert Null ein geeigneter Schwellwert ist, sodass die Generierung des Steuersignals einfach zu handhaben ist. Außerhalb des fraglichen Intervalls für die Signalwerte des Elektromyogramm-Signals um den Basiswert kann die Bewertungsfunktion derart definiert sein, dass hier ein konstanter Wert oder zunächst linear ansteigende Werte und dann ein konstanter Wert verwendet wird.

Schließlich ist in diesem Zusammenhang noch anzumerken, dass es bei der Durchführung des erfindungsgemäßen Verfahrens auch möglich ist, dass vor dem Schritt des Transformierens das Elektromyogramm-Signal in der Weise bearbeitet wird, dass ein konstanter Untergrund abgezogen wird, damit das so bearbeitete Signal anschließend um Null variiert. In einem solchen Fall oder dem Fall, dass das Elektromyogramm-Signal von sich aus um Null variiert, ist der Basiswert in diesem Zusammenhang gleich Null.

Wie schon erwähnt wird der Hauptparametersatz kontinuierlich neu bestimmt und damit während des Durchlaufs des Verfahrens bzw. des Betriebs der Vorrichtung immer wieder neu an die Bedingungen angepasst.

Bei der erfindungsgemäßen Vorrichtung sind dann die entsprechenden, in Anspruch 1 aufgeführten Mittel vorgesehen, nämlich:
- Mittel zum Transformieren des Elektromyogramm-Signals (E) in ein Bewertungssignal (F, G) durch Anwenden einer Bewertungsfunktion auf das Elektromyogramm-Signal (E), wobei beim Transformieren einem Signalwert (x) des Elektromyogramm-Signals (E) ein Signalwert (y) des Bewertungssignals (F, G) zugewiesen wird,
   wobei die Bewertungsfunktion durch einen Hauptparametersatz bestimmt ist, sodass der Hauptparametersatz festlegt, welchem Signalwert (x) des Elektromyogramm-Signals (E) beim Transformieren bei Anwendung der Bewertungsfunktion welcher Signalwert (y) des Bewertungssignals (F, G) zugewiesen wird, und
   wobei die Höhe des Signalwerts (y) des Bewertungssignals (F, G) angibt, ob das Elektromyogramm-Signal (E) einer Inspirationsphase oder einer Exspirationsphase zuzuordnen ist, wobei bei Anwendung der Bewertungsfunktion auf Signalwerte (x) aus einem Intervall um einen Basiswert ein Signalwert (y) des Bewertungssignals (F, G) zugewiesen wird und die Bewertungsfunktion der Logarithmus des Verhältnisses der Wahrscheinlichkeiten P_{I}(x), P_{E}(x) ist, dass der Signalwert (x) des Elektromyogramm-Signals (E) zu der Inspirationsphase oder zu der Exspirationsphase gehört,
- Mittel zum Generieren eines Steuersignals (H) aus zeitlich aufeinander folgenden Signalwerten, das angepasst ist, ein Beatmungsgerät (7) in eine Inspirationsbetriebsart zu schalten, wenn das Bewertungssignal (F, G) der Inspirationsphase entspricht, und in eine Exspirationsbetriebsart zu schalten, wenn das Bewertungssignal (F, G) der Exspirationsphase entspricht, und
- Mittel, die, parallel zum Transformieren des Elektromyogramm-Signals (E) und dem Generieren des Steuersignals (H), zum Analysieren zumindest eines Teils des Elektromyogramm-Signals als dessen Analyseabschnitt mit folgenden Mitteln zur Anpassung des Hauptparametersatzes eingerichtet sind:
   - Mitteln zum Transformieren eines Analyseabschnitts (E') des Elektromyogramm-Signals (E) in ein zweites Bewertungssignal (F', G') durch Anwenden der Bewertungsfunktion auf den Analyseabschnitt (E'), wobei beim Transformieren einem Signalwert (X) des Analyseabschnitts (E') ein Signalwert (Y) des zweiten Bewertungssignals (F', G') zugewiesen wird,
      wobei die Bewertungsfunktion durch einen Analyseparametersatz bestimmt ist, sodass der Analyseparametersatz festlegt, welchem Signalwert (X) des Analyseabschnitts (E') beim Transformieren bei Anwendung der Bewertungsfunktion welcher Signalwert (Y) des zweiten Bewertungssignals (F', G') zugewiesen wird, und
      wobei die Höhe des Signalwerts (Y') des zweiten Bewertungssignals (F', G') angibt, ob der korrespondierende Signalwert (X) des Analyseabschnitts (E') einer Inspirationsphase oder einer Exspirationsphase entspricht,
   - Mitteln zum Bestimmen einer ersten Häufigkeitsverteilung H₁(X) für die Signalwerte (X) aus den Teilen des Analyseabschnitts (E'), für die das zweite Bewertungssignal (F', G') angibt, dass die Inspirationsphase vorliegt,
   - Mitteln zum Bestimmen einer zweiten Häufigkeitsverteilung H₂(X) für die Signalwerte (X) aus den Teilen des Analyseabschnitts (E'), für die das zweite Bewertungssignal (F', G') angibt, dass die Exspirationsphase vorliegt,
   - Mitteln zum Bestimmen eines erneuerten Parametersatzes aus den Häufigkeitsverteilungen H₁(X), H₂(X) und
   - Mitteln zum Übergeben des erneuerten Parametersatzes als Hauptparametersatz,
      wobei die Mittel zum Bestimmen der ersten und zweiten Häufigkeitsverteilung H₁(X), H₂(X), die Mittel zum Bestimmen des erneuerten Parametersatzes und die Mittel zum Übergeben des erneuerten Parametersatzes ausgestaltet sind, parallel zu den Mittel zum Transformieren des Elektromyogramm-Signals (E) und denen zum Generieren des Steuersignals (H) zu arbeiten.

Demnach wird parallel zu der Transformation des Elektromyogramm-Signals und zu dem Generieren des Steuersignals der der Transformation zugrundeliegende Hauptparametersatz in der Weise bestimmt, dass auch hierfür das Elektromyogramm-Signal, und zwar ein Abschnitt davon, nämlich der Analyseabschnitt, zunächst in ein zweites Bewertungssignal transformiert wird. Dann werden in dem Analyseabschnitt diejenigen Abschnitte bestimmt, in denen der erste Zustand, also die Inspirationsphase, vorliegt, und diejenigen Abschnitte, in denen der zweite Zustand, also die Exspirationsphase, gegeben ist. Schließlich werden die Abschnitte separat analysiert.

Dies geschieht in der Weise, dass die schon beschriebene Bewertungsfunktion, die in diesem Fall durch einen Analyseparametersatz bestimmt ist, auf die Signalwerte des Analyseabschnitts angewandt wird. Hierbei wird vorzugsweise für die Bewertungsfunktion ein Standardparametersatz oder der gerade aktuelle Hauptparametersatz als Analyseparametersatz verwendet. Dabei kann ein Standardparametersatz in der Weise ermittelt werden, dass zunächst die Magnitude des Eingangssignals über einen Zeitraum bestimmt wird, der deutlich größer als der Kehrwert der Atemfrequenz ist. Diese Magnitude wird stark geglättet und anschließend mit dem Eingangssignal verglichen. Der Standardparametersatz wird nun so festgelegt, dass Signalwerte mit einer Magnitude unterhalb der geglätteten Magnitude der Exspirationsphase zugeordnet werden, während Signalwerte mit einer Magnitude oberhalb der geglätteten Magnitude der Inspirationsphase zugeordnet werden.

Anschließend werden in weiter bevorzugter Weise diejenigen Signalwerte des Analyseabschnitts bestimmt, für die der Signalwert des Bewertungssignals oberhalb einer Schwelle liegt, und diejenigen, für die der Signalwert unterhalb der Schwelle liegt.

Die Signalwerte des Analyseabschnitts, bei denen der entsprechende Signalwert des Bewertungssignals die Schwelle überschreitet, werden als zu dem ersten Zustand, nämlich der Inspirationsphase, gehörig markiert, während diejenigen Signalwerte des Analyseabschnitts, bei denen die Signalwerte des zweiten Bewertungssignals unterhalb der Schwelle liegen, als zu dem zweiten Zustand, also der Exspirationsphase, zugehörig markiert werden.

Anschließend wird separat für diejenigen Signalwerte des Analyseabschnitts, die als zu dem ersten Zustand und damit der Inspirationsphase zugehörig markiert wurden, eine Auswertung durchgeführt, bei der eine erste Häufigkeitsverteilung für die Signalwerte erstellt wird. Diese erste Häufigkeitsverteilung ist dann proportional zu der Wahrscheinlichkeit P_{I}(x), dass ein Signalwert x zu der Inspirationsphase gehört. In gleicher Weise wird für diejenigen Signalwerte, die als zu dem zweiten Zustand, also der Exspirationsphase, zugehörig markiert worden sind, eine zweite Häufigkeitsverteilung erstellt, die dann ein Maß für die Wahrscheinlichkeit P_{E}(x) angibt, dass ein Signal x zu der Exspirationsphase gehört.

Aus der ersten und zweiten Häufigkeitsverteilung wird dann ein erneuerter Parametersatz bestimmt, wobei dies in besonders bevorzugter Weise dadurch erfolgt, dass der erneuerte Parametersatz als Tabelle aufgebaut ist, die jedem möglichen Signalwert des Elektromyogramm-Signals einen Signalwert des zweiten Bewertungssignals zuweist, wobei für einen Signalwert y des Elektromyogramm-Signals, der in einem Intervall um einen Basiswert liegt, der zugewiesene Signalwert der Tabelle eine Funktion des Verhältnisses des Wertes der ersten Häufigkeitsverteilung für den Signalwert x zu dem Wert der zweiten Häufigkeitsverteilung für den Signalwert x ist. Wenn bei dem Elektromyogramm-Signal vor der weiteren Verarbeitung zunächst ein konstanter Untergrund abgezogen wird, ist der Basiswert auch hier Null. Bei der Funktion des Quotienten handelt es sich aus den bereits beschriebenen Gründen um den Logarithmus.

Schließlich kann die Tabelle in weiter bevorzugter Weise als zugewiesenen Signalwert für Signalwerte x des Analyseabschnitts bzw. des Elektromyogramm-Signals aus einem Randintervall, das außerhalb des Intervalls liegt, einen konstanten Wert enthalten.

Außerdem kann in weit vom Basiswert entfernten Bereichen, in denen aufgrund von sehr seltenen Eintrittswahrscheinlichkeiten auch nur ungenaue Schätzungen der Häufigkeitsverteilungen vorliegen, der Parametersatz durch vereinfachte Funktionsverläufe, z.B. Konstanten, lineare Funktionen oder andere einfache Funktionen ersetzt bzw. abgeschätzt werden.

Wenn dieser Prozess für einen vorgegebenen Analyseabschnitt abgeschlossen ist, wird der so erstellte erneuerte Parametersatz als Hauptparametersatz entsprechend übergeben und verwendet.

Diese Vorgehensweise schafft die Möglichkeit, kontinuierlich den Hauptparametersatz, der bei der Erzeugung des Steuersignals verwendet wird, immer weiter zu optimieren oder an Veränderungen des Patienten anzupassen, die sich beispielsweise dadurch ergeben können, dass sich das Elektromyogramm-Signal durch eine Bewegung des Patienten ändert bzw. verschiebt.

Daher ist es auch, wie schon erwähnt, bevorzugt, dass bei der Bestimmung des Parametersatzes in einem Analyseabschnitt des Elektromyogramm-Signals zunächst der Hauptparametersatz für die Bewertungsfunktion und die Zuordnung der einzelnen Teile des Analyseabschnitts zur Exspirations- bzw. Inspirationsphase verwendet wird. Somit wird dann eine iterative Vorgehensweise implementiert.

Schließlich kann die Zuordnung der Signalwerte des Analyseabschnitts zu dem ersten und dem zweiten Zustand, also der Inspirationsphase und der Exspirationsphase, durch ein Analysesignal erfolgen. Dabei weist die Vorrichtung vorzugsweise entsprechende Mittel zur Erzeugung eines solchen Analysesignals auf. Dieses Analysesignal, das für jeden Signalwert des Analyseabschnitts einen Analysesignalwert enthält, wird in der Weise generiert, dass ein Analysesignalwert einen ersten Wert annimmt, wenn derjenige Signalwert des zweiten Bewertungssignals, der dem Signalwert des Analyseabschnitts entspricht, zu dem der Analysesignalwert generiert wird, oberhalb eines Schwellwerts liegt, und dass ein Analysesignalwert einen zweiten Wert annimmt, wenn derjenige Signalwert des zweiten Bewertungssignals, der dem Signalwert des Analyseabschnitts entspricht, zu dem der Analysesignalwert generiert wird, unterhalb eines Schwellwerts liegt. Die erste Häufigkeitsverteilung wird für die Signalwerte des Analyseabschnitts bestimmt, für die der Analysesignalwert den ersten Wert hat, und die zweite Häufigkeitsverteilung wird für die Signalwerte des Analyseabschnitts bestimmt, für die der Analysesignalwert den zweiten Wert hat.

In weiter bevorzugter Weise wird bei der Bestimmung des Parametersatzes das zweite Bewertungssignal geglättet, wobei hier eine Mittelung über eine im Vergleich zu der Glättung bei der Bestimmung des Steuersignals größere zweite Anzahl von Signalwerten erfolgt. Ansonsten entspricht die Vorgehensweise beim Glätten der, wie sie schon im Zusammenhang mit der Glättung des Elektromyogramm-Signals und des ersten Bewertungssignals bei der Generierung des Steuersignals beschrieben wurde.

Allerdings führt dann die größere zweite Anzahl von Signalwerten, die bei der Glättung berücksichtigt werden, dazu, dass die Bestimmung des Parametersatzes vergleichsweise langsam erfolgt. Dies ist aber keineswegs nachteilhaft, wobei zu berücksichtigen ist, dass durch die größere Glättung auch eine höhere Genauigkeit bei der Bestimmung der Häufigkeitsverteilungen erzielt wird.

Schließlich ist es bei der Bestimmung des Parametersatzes bevorzugt, die Einteilung, die festlegt, welche Teile des Analyseabschnitts zur Inspirationsphase und welche zur Exspirationsphase gehören, mit einem Analysesignal durchzuführen, das ähnlich dem Steuersignal binär ist und so die einzelnen Teile kennzeichnet.

Im Folgenden wird die vorliegende Erfindung anhand einer Zeichnung erläutert, die lediglich ein bevorzugtes Ausführungsbeispiel wiedergibt, wobei
- Fig. 1: ein Flussdiagramm des Ausführungsbeispiels eines hierin beschriebenen Verfahrens, nach dem das Ausführungsbeispiel einer erfindungsgemäßen Vorrichtung betrieben wird, zeigt,
- Fig. 2: einen Teil des Flussdiagramms aus Fig. 1 im Detail zusammen mit den dabei erzeugten Signale als Funktion der Zeit zeigt,
- Fig. 3: eine schematische Darstellung des Ablaufs des Verfahrens zusammen mit den entsprechenden Signalen als Funktion der Zeit ist und
- Fig. 4: eine schematische Darstellung eines Ausführungsbeispiels einer erfindungsgemäßen Vorrichtung zum Erzeugen eines Steuersignals zeigt.

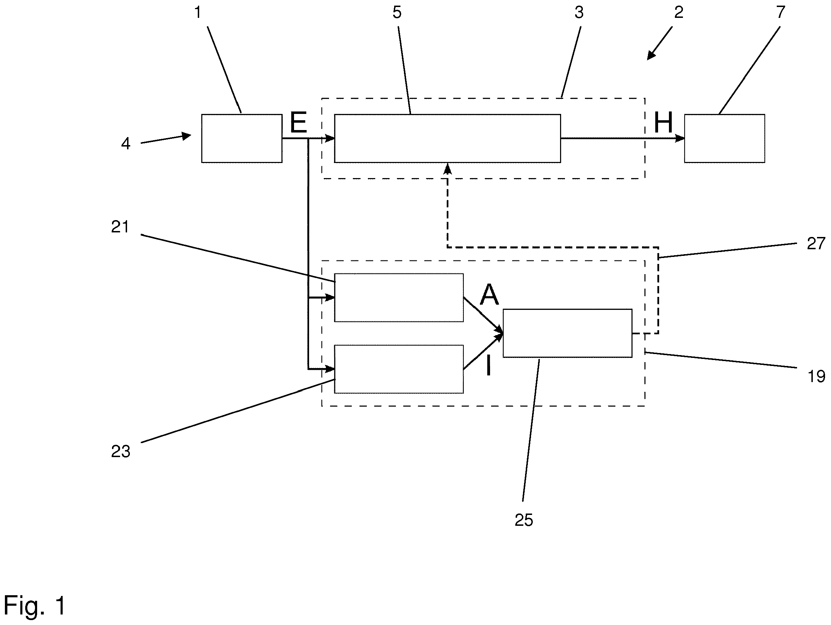

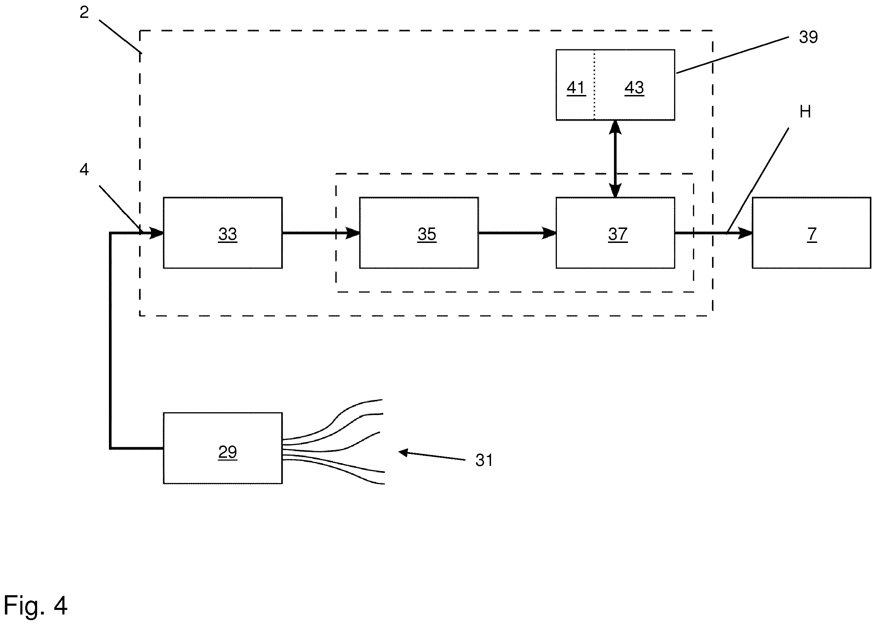

Anhand der Zeichnung ist ein Ausführungsbeispiel eines hierin beschriebenen Verfahrens im Detail erläutert. Dabei zeigt Fig. 1 neben einem schematischen Flussdiagramm des gesamten Verfahrens gemäß dem bevorzugten Ausführungsbeispiel auch schematisch den Aufbau einer entsprechenden Vorrichtung 2 zum Erzeugen eines Steuersignals, die mit den entsprechenden Mitteln zum Durchführen der Verfahrensschritte ausgestattet ist, wobei ein Ausführungsbeispiel einer solchen Vorrichtung 2 auch in Fig. 4 wiedergegeben ist.

Wie weiter aus Fig. 1 zu erkennen ist, wird bei diesem Ausführungsbeispiel zunächst in einem ersten Erfassungsschritt 1 ein Elektromyogramm-Signal eines Patienten aufgenommen. Dieses Signal kann in an sich bekannter Weise mit Hilfe von auf der Haut des Patienten im Bereich des Brustkorbs angebrachter Elektroden erfasst werden.

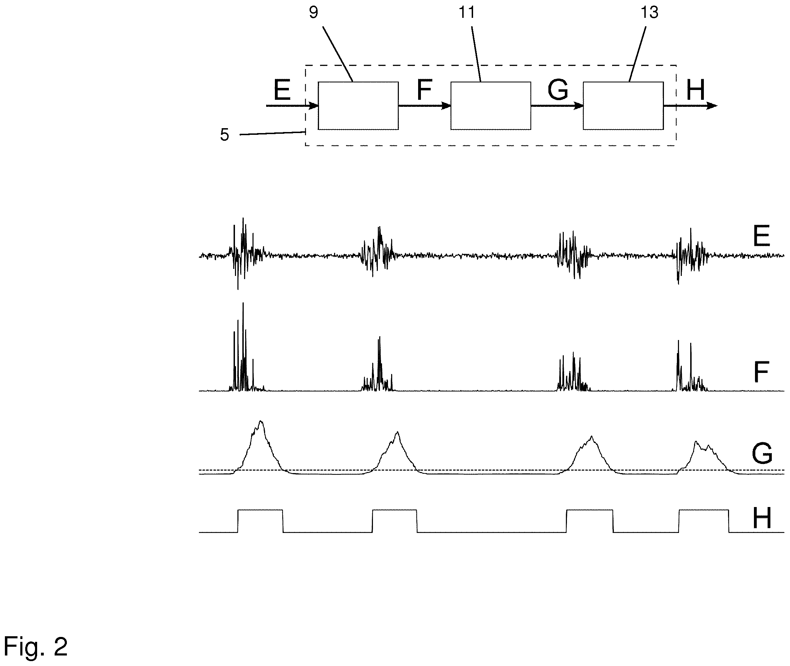

Bevor das Signal weiterverarbeitet wird, kann in dem Erfassungsschritt 1 noch ein konstanter oder zeitlich nur langsam variierender Untergrund von dem Elektromyogramm-Signal abgezogen werden, sodass sich das dann ergebende Signal im Wesentlichen um einen Signalwert von Null variiert. Ein derartiges Elektromyogramm-Signal ist in Fig. 2 mit "E" gekennzeichnet.

Das so erhaltene und von einem möglichen Untergrund befreite Elektromyogramm-Signal E wird einem sogenannten Steuersignalpfad 3 oder Hauptsegmentierungspfad zugeführt, wobei in der Vorrichtung 2 ein Signaleingang 4 vorgesehen ist, über den das Rohsignal vor einer möglichen Korrektur des Untergrundes der Vorrichtung 2 zugeführt wird.

Der Steuersignalpfad 3 umfasst einen Schritt 5 zum Erzeugen eines Steuersignals für ein Beatmungsgerät 7, das mit einem Patienten verbunden sein kann. Dieser Schritt 5 des Erzeugens des Steuersignals ist in Fig. 2 im Detail dargestellt, wobei das hier ausgegebene Steuersignal H geeignet ist, das Beatmungsgerät 7 zwischen einer Inspirationsbetriebsart, in der einem Patienten Atemgas aktiv zugeführt wird, und einer Exspirationsbetriebsart hin und her zu schalten. Dazu muss aus dem Elektromyogramm-Signal E bestimmt werden, ob dessen jeweiliger Signalwert x einerseits einem ersten Zustand des Patienten entspricht, in dem dieser Atemanstrengungen unternimmt und daher die Potentiale an den atmungsrelevanten Muskeln erhöht sind, also die Inspirationsphase vorliegt. Andererseits kann bei dem Patienten auch ein zweiter Zustand vorliegen, in dem er keine Atemanstrengungen unternimmt, also die Exspirationsphase gegeben ist, was sich in verminderten Potentialen an den atmungsrelevanten Muskeln widerspiegelt.

Das sich dabei ergebende Steuersignal ist in Fig. 2 mit "H" gekennzeichnet, und im Folgenden wird erläutert, wie dieses Steuersignal H bei dem hierin beschriebenen Verfahren im Steuersignal- oder Hauptsegmentierungspfad 3 im Schritt 5 generiert wird.

Wie aus Fig. 2 zu erkennen ist, wird im Schritt 5 als Erstes ein Transformationsschritt 9 durchgeführt, beim dem das Elektromyogramm-Signal E in ein Bewertungssignal F durch Anwenden einer Bewertungsfunktion auf das Elektromyogramm-Signal E umgewandelt wird. Bei diesem Transformationsschritt 9 wird jedem Signalwert x des Elektromyogramm-Signals E ein Signalwert y des Bewertungssignals F zugewiesen, wobei die Bewertungsfunktion durch einen Hauptparametersatz bestimmt ist, sodass der Hauptparametersatz festlegt, welchem Signalwert x des Elektromyogramm-Signals E beim Transformieren bei Anwendung der Bewertungsfunktion welcher Signalwert y des Bewertungssignals F zugewiesen wird.

In dem hier beschriebenen bevorzugten Ausführungsbeispiel liegt der Hauptparametersatz als Tabelle vor, in der für jeden möglichen Signalwert x des Elektromyogramm-Signals E der zugewiesene Signalwert y für das Bewertungssignal F hinterlegt ist. Es ist aber auch denkbar, dass der Hauptparametersatz die Parameter der mathematischen Funktion festlegt, mit der aus dem Signalwert x des Elektromyogramm-Signals der entsprechende Signalwert y des Bewertungssignals berechnet wird.

Die in der Tabelle für die Signalwerte x des Elektromyogramm-Signals E enthaltenen Signalwerte y des Bewertungssignals F ergeben sich in diesem bevorzugten Ausführungsbeispiel in einem Intervall um einen Basiswert in der Weise, dass sie eine Funktion des Verhältnisses der Wahrscheinlichkeit P_{I}(x), dass der Signalwert x dem Inspirationszustand entspricht, zu der Wahrscheinlichkeit P_{E}(x), dass der Signalwert x dem Exspirationszustand entspricht, ist.

Diese Funktion ist der Logarithmus, was mit dem Effekt verbunden ist, dass dann, wenn die Wahrscheinlichkeit P_{I}(x), dass die Inspirationsphase vorliegt, größer ist als die Wahrscheinlichkeit P_{E}(x), dass die Exspirationsphase vorliegt, der Signalwert y der Bewertungsfunktion größer Null ist. Umgekehrt ist dann, wenn die Wahrscheinlichkeit P_{E}(x), dass die Exspirationsphase vorliegt, größer ist als die Wahrscheinlichkeit P_{I}(x), dass die Inspirationsphase vorliegt, der Signalwert y der Bewertungsfunktion kleiner Null. Damit ergibt sich später grundsätzlich eine einfache Möglichkeit, durch eine Schwellwertanalyse zu bestimmen, welche der beiden Phasen bzw. welcher der beiden möglichen Zustände vorliegt.

Wenn im Transformationsschritt 9 die Bewertungsfunktion auf das Elektromyogramm-Signal angewandt wird, wird einem Signalwert x des Elektromyogramm-Signals E der der Tabelle entsprechende Wert als Signalwert y des Bewertungssignals F zugewiesen, sodass sich zunächst das in Fig. 2 gezeigte Bewertungssignal F ergibt.

In dem hier beschriebenen bevorzugten Ausführungsbeispiel wird nach dem Anwenden der Bewertungsfunktion das so erzeugte Bewertungssignal F in einem Glättungsschritt 11 dadurch geglättet, dass jeder Signalwert y des Bewertungssignals F als vorgegebener Signalwert y' behandelt wird und dass dann jeder vorgegebene Signalwert y' des Bewertungssignals F ersetzt wird durch einen Mittelwert aus Signalwerten des Bewertungssignals F, die den vorgegebenen Signalwert umfassen können und eine erste Anzahl von zeitlich vor dem vorgegebenen Signalwert y' liegende Signalwerte y umfassen. Es wird also über ein Fenster, das mitläuft, gemittelt.

Auch hier sollen unter den Begriff "Mittelwert" im Sinne der vorliegenden Anmeldung nicht nur das arithmetische Mittel fallen, sondern auch Werte, die unter Anwendung einer Gewichtungsfunktion gewonnen werden. Außerdem fallen auch solche Werte darunter, die durch Anwendung nichtlinearer Glättungsmethoden wie Rangordnungsfilter oder Medianfilter bestimmt werden.

Dies bedeutet, dass die Signalwerte y des Bewertungssignals F in der Weise bearbeitet werden, dass sie in der Reihenfolge, in der für sie die entsprechenden Elektromyogramm-Signalwerte x erfasst wurden, als vorgegebene Signalwerte y' behandelt werden. Für jeden vorgegebenen Signalwert y' wird dann aus einer ersten Anzahl von zeitlich vor dem vorgegebenen Signalwert y' erfassten Signalwerten y ein Mittelwert gebildet und der vorgegebene Signalwert y' durch diesen Mittelwert ersetzt. Das bedeutet, dass die erste Anzahl von Signalwerten aus Werten besteht, für die die zuvor beschriebene Mittelwertbildung schon durchgeführt wurde. Das so im Glättungsschritt 11 geglättete Bewertungssignal G ist ebenfalls in Fig. 2 gezeigt.

In der hier beschriebenen bevorzugten Ausführungsform des Verfahrens wird der Mittelwert in der Weise gebildet, dass zeitlich nahe an dem vorgegebenen Signalwert y' liegende Werte stärker gewichtet in die Mittelwertbildung eingehen als solche aus der ersten Anzahl von Signalwerten, die zeitlich weiter vor dem vorgegebenen Signalwert y' liegen. Dadurch ergibt sich der Vorteil, dass trotz der Mittelwertbildung der Glättungsschritt 11 nicht dazu führt, dass Änderungen des Bewertungssignals selbst durch den Glättungsschritt 11 zeitlich stark nach hinten verschoben werden. Im Übrigen ist darauf hinzuweisen, dass der Begriff des Mittelwerts im Sinne der vorliegenden Erfindung nicht allein das arithmetische Mittel beschränkt ist, sondern auch andere gewichtete Mittel umfasst. Außerdem sollen unter den Begriff "Mittelwert" auch solche Werte fallen, die durch Anwendung nichtlinearer Glättungsmethoden wie Rangordnungsfilter oder Medianfilter gewonnen werden.

Wie Fig. 2 zeigt, gibt die Höhe des Signalwerts y' des geglätteten Bewertungssignals G dann an, ob das Elektromyogramm-Signal E einem ersten Zustand oder einen zweiten Zustand entspricht, also die Inspirationsphase oder die Exspirationsphase vorliegt.

Nach dem Durchführen des Glättungsschritts 11 erfolgt innerhalb des Steuersignalpfades 3 während des Schrittes 5 zur Erzeugung des Steuersignals das eigentliche Generieren eines Steuersignals. Dies erfolgt bei dem hier beschriebenen Ausführungsbeispiel durch einen Schwellwertvergleichsschritt 13, bei dem die Signalwerte y' des geglätteten Bewertungssignals G mit dem Schwellwert Null verglichen werden, wie dies auch in Fig. 2 eingezeichnet ist. Der Schwellwert von Null ergibt sich aus der Wahl des Logarithmus als der Funktion des Quotienten aus den Wahrscheinlichkeiten P_{I}(x) und P_{E}(x) dafür, dass ein Signalwert des Elektromyogramm-Signals zu der Inspirations- oder der Exspirationsphase bei dem Patienten gehört.

Somit wird beim Generieren des Steuersignals jeder Signalwert y' des in diesem Fall geglätteten Bewertungssignals G mit einem Schwellwert von Null verglichen, wobei das in Fig. 2 gezeigte Steuersignal H einen ersten, dem ersten Zustand entsprechenden Steuerwert annimmt, wenn der Signalwert y' des geglätteten Bewertungssignals G oberhalb des Schwellwerts liegt, und wobei das Steuersignal H einen zweiten, dem zweiten Zustand entsprechenden Steuerwert annimmt, wenn der Signalwert y' des geglätteten Bewertungssignals G unterhalb des Schwellwerts liegt. Das Steuersignal H ist dann aus zeitlich aufeinander folgenden Signalwerten gebildet und angepasst, ein Beatmungsgerät in eine Inspirationsbetriebsart zu schalten, wenn das Bewertungssignal dem ersten Zustand, also der Inspirationsphase, entspricht, und in eine Exspirationsbetriebsart zu schalten, wenn das Bewertungssignal dem zweiten Zustand, also der Exspirationsphase, entspricht.

Wenn das Elektromyogramm-Signal E Abschnitte enthält, für die eine Auswertung nicht möglich ist, beispielsweise weil ein EKG-Signal überlagert ist, gibt es in bevorzugter Weise die folgenden Möglichkeiten, wie damit umgegangen wird.

Zunächst kann der ganze Steuersignalpfad 3 pausieren, bis wieder auswertbare oder gültige Daten am Eingang erfasst werden. Es ist auch möglich, dass bei ungültigen oder nichtauswertbaren Abschnitten des Elektromygramm-Signals E das Bewertungssignal F auf 0 gesetzt wird. Der Nachteil hierbei besteht darin, dass nach längeren Abschnitten mit ungültigen Werten das erste wieder gültige Signal allein die Entscheidung über darüber, ob das Steuersignal H den einen oder anderen Wert annimmt. Als weitere Alternative kann der Steuersignalpfad 3 pausieren, bis wieder gültige Daten am Eingang anliegen. In der Zwischenzeit werden die veralteten Werte des Bewertungssignals F schrittweise und bis zu einem bestimmten Grad aus dem Glättungsfilter entfernt. Schließlich kann bei nichtauswertbaren Abschnitten des Elektromyogramm-Signals E künstliche Bewertungssignalwerte erzeugt werden, die in der Nähe des neutralen Bereichs des Bewertungssignals F, also bei Null, liegen. Bei diesen Werten handelt es sich um betragsmäßig kleine Werte, deren Vorzeichen der zuletzt detektierten Hypothese entspricht. Die letzten beiden Möglichkeiten sind vorteilhaft, weil nicht der erste wieder gültige Elektromyogramm-Signalwert zwingend über einen Sprung im Steuersignal H entscheidet und andererseits ein Sprung nicht durch viele alte Bewertungssignalwerte im Glättungsfilter verzögert wird.

Parallel zu dem Steuersignalpfad 3 wird zumindest ein Teil des zuvor von einem konstanten Untergrund befreiten Elektromyogramm-Signals E in einem Nebensegmentierungs- oder Analysepfad 19 verarbeitet, um den in dem Steuersignalpfad 3 in der Bewertungsfunktion verwendeten Hauptparametersatz zu ermitteln. Unter "parallel" ist in diesem Zusammenhang zu verstehen, dass die Verarbeitung in dem Analysepfad 19 zeitgleich mit der Erzeugung des Steuersignals H in dem Steuersignalpfad 3 abläuft.

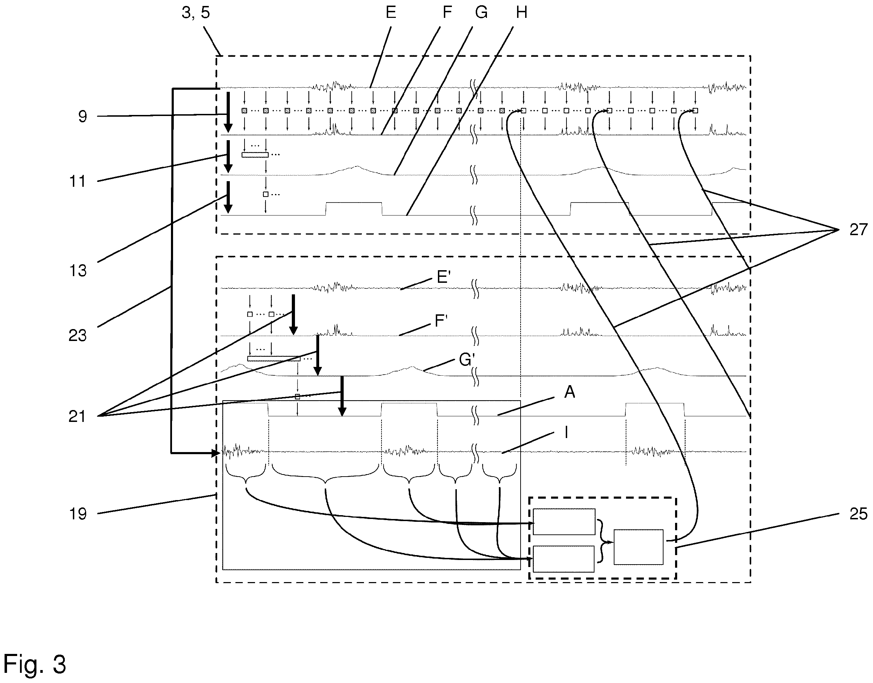

In dem Analyse- oder Nebensegmentierungspfad 19 wird ein Analyseabschnitt E' (siehe Fig. 3), der ein Teil des vom Untergrund befreiten Elektromyogramm-Signals E ist, im vorliegenden Ausführungsbeispiel in einem ersten Analyseschritt 21 zunächst mit der Bewertungsfunktion analysiert. Dazu wird der Analyseabschnitt E' in ein zweites Bewertungssignal F' durch Anwenden der Bewertungsfunktion auf den Analyseabschnitt E' transformiert, wobei beim Transformieren einem Signalwert des Analyseabschnitts E' ein Signalwert Y des zweiten Bewertungssignals F' zugewiesen wird. In diesem Fall ist die Bewertungsfunktion durch einen Analyseparametersatz bestimmt ist, sodass der Analyseparametersatz festlegt, welchem Signalwert X des Analyseabschnitts E' beim Transformieren bei Anwendung der Bewertungsfunktion welcher Signalwert Y des zweiten Bewertungssignals F' zugewiesen wird.

In dem hier beschriebenen bevorzugten Ausführungsbeispiel wird als Analyseparametersatz der auch im Hauptsegmentierungs- oder Steuersignalpfad 3 im Schritt 5 verwendete Hauptparametersatz verwendet. Es ist aber auch denkbar, einen anderen Parametersatz im Analysepfad 19 anzuwenden. Außerdem liegen der Analyseparametersatz und der später bestimmte erneuerte Parametersatz als Tabelle vor, sodass die Tabelle des Hauptparametersatzes jedem möglichen Signalwert X des Analyseabschnitts E' einen Signalwert Y des zweiten Bewertungssignals F' zuweist.

Nach dem Transformieren des Analyseabschnitts E' wird das so erzeugte zweite Bewertungssignal F' dadurch geglättet, dass jeder Signalwert Y des zweiten Bewertungssignals F', wie schon im Zusammenhang mit Schritt 11 erläutert, als vorgegebener Signalwert Y' behandelt wird und jeder vorgegebene Signalwert Y' dann ersetzt wird durch einen Mittelwert aus Signalwerten Y' des zweiten Bewertungssignals F', die den vorgegebenen Signalwert Y' umfassen können und die eine zweite Anzahl von zeitlich vor dem vorgegebenen Signalwert Y' liegende Signalwerte Y' umfassen, wobei diese zweite Anzahl jedoch größer als die im Schritt 11 verwendete erste Anzahl ist. Dadurch, dass die zweite Anzahl größer als die erste Anzahl ist, wird eine stärkere Glättung erreicht, was die Genauigkeit der Analyse im Analysepfad 19 erhöht. Allerdings ist dies damit verbunden, dass das geglättete zweite Bewertungssignal G' (siehe Fig. 3) sich bei Änderungen der Signalwerte des Analyseabschnitts E' langsamer ändert, als dies das im Steuersignalpfad 3 in Schritt 5 bestimmte Bewertungssignal F tut.

Das so geglättete zweite Bewertungssignal G' ist dadurch so bearbeitet, dass die Höhe eines Signalwerts Y' dieses geglätteten zweiten Bewertungssignals G' angibt, ob der korrespondierende Signalwert X des Analyseabschnitts E' einem ersten Zustand oder einen zweiten Zustand entspricht, also zu der Inspirationsphase oder zu der Exspirationsphase gehört. Schließlich wird in dem ersten Analyseschritt 21 noch ein Analysesignal I (siehe Fig. 3) aus dem geglätteten zweiten Bewertungssignal G' generiert, das für jeden Signalwert X des Analyseabschnitts E' einen Analysesignalwert A enthält. Der Analysesignalwert A nimmt einen ersten Wert an, wenn derjenige Signalwert Y' des geglätteten zweiten Bewertungssignals G', der dem Signalwert X des Analyseabschnitts E' entspricht, zu dem der Analysesignalwert A generiert werden soll, oberhalb eines Schwellwerts liegt. Bei diesem Schwellwert handelt es sich um den Wert Null, da der Bewertungsfunktion und dem Analyse- bzw. dem Hauptparametersatz die Funktion lg(P_{I}(X)/P_{E}(X)) zugrunde liegt, wobei P_{I}(X) und P_{E}(X) die Wahrscheinlichkeiten angeben, dass ein Signalwert X des Analyseabschnitts E' dem ersten Zustand, also der Inspirationsphase, oder dem zweiten Zustand, d.h. der Exspirationsphase, entspricht.

Der Analysesignalwert A nimmt dann einen zweiten Wert an, wenn derjenige Signalwert Y' des geglätteten zweiten Bewertungssignals G', der dem Signalwert X des Analyseabschnitts E' entspricht, zu dem der Analysesignalwert A generiert wird, unterhalb dieses Schwellwerts liegt.

Im parallel zum ersten Analyseschritt 21 ablaufenden Verzögerungsschritt 23 wird das den Analyseabschnitt E' darstellende Signal so verzögert, dass es parallel mit dem im ersten Analyseschritt 21 erzeugten Analysesignal A im zweiten Analyseschritt 25 verarbeitet werden kann. Das bedeutet, dass das verzögerte Signal I des Analyseabschnitts E' so mit dem eingehenden Analysesignal A zusammenfällt, dass ein Signalwert X des Analyseabschnitts E' für den im ersten Analyseschritt 21 der Analysesignalwert A bestimmt worden ist, mit diesem Analysesignalwert A zusammen im zweiten Analyseschritt 25 verarbeitet werden kann. Insbesondere kann dann der Analysesignalwert A unmittelbar für den Signalwert X des Analyseabschnitts E' anzeigen, ob dieser Signalwert X zu der Inspirations- oder zu der Exspirationsphase gehört.

In dem zweiten Analyseschritt 25 wird dann eine erste Häufigkeitsverteilung H₁(X) für die Signalwerte X des Analyseabschnitts bestimmt, für die das Analysesignal A den ersten Wert annimmt und für die das geglättete zweite Bewertungssignal G' somit angibt, dass der erste Zustand, also der Inspirationszustand, vorliegt. Außerdem wird parallel im zweiten Analyseschritt 25 auch eine zweite Häufigkeitsverteilung H₂(X) für die Signalwerte X des Analyseabschnitts E' bestimmt, für die das Analysesignal A den zweiten Wert annimmt und das zweite Bewertungssignal G' dafür somit angibt, dass der zweite Zustand, also der Exspirationszustand, vorliegt.

Schließlich wird im zweiten Analyseschritt 25 ein erneuerter Parametersatz aus den zuvor bestimmten Häufigkeitsverteilungen H₁(X), H₂(X) bestimmt. Der erneuerte Parametersatz ist als Tabelle aufgebaut, die jedem möglichen Signalwert des Elektromyogramm-Signals E einen Signalwert des Bewertungssignals zuweist.

Dabei ist für einen Signalwert des Elektromyogramm-Signals, der in einem Intervall um einen Basiswert liegt, der zugewiesene Signalwert der Tabelle eine Funktion des Verhältnisses des Wertes der ersten Häufigkeitsverteilung H₁(x) für den Signalwert x zu dem Wert der zweiten Häufigkeitsverteilung H₂(x) für den Signalwert x. Insbesondere ist der zugewiesene Signalwert y proportional zu dem Logarithmus des Quotienten der Häufigkeitsverteilungen, sodass für die in der Tabelle für den Signalwert x enthaltenen Werte y gilt, dass y ∝ lg(H₁(x)/H₂(x)) ist. Dadurch, dass bei dem hier beschriebenen Ausführungsbeispiel ein sich zeitlich im Vergleich zum Elektromyogramm-Signal langsam verändernder Untergrund vom Elektromyogramm-Signal abgezogen wird, ist der Basiswert hier Null.

Außerdem ist anzumerken, dass die Häufigkeitsverteilung H₁(x) ein direktes Maß für die Wahrscheinlichkeit P_{I}(x) ist, dass der Signalwert x zu der Inspirationsphase gehört, d.h. dem ersten Zustand entspricht, während die Häufigkeitsverteilung H₂(x) ein Maß für die Wahrscheinlichkeit P_{E}(x) ist, dass der Signalwert x zu der Exspirationsphase gehört, d.h. dem zweiten Zustand entspricht.

In den beiden Randintervallen, die außerhalb des Intervalls um den Basiswert liegen, hat die Tabelle für die entsprechenden Signalwerte x des Elektromyogramm-Signals einen konstanten, ggf. für jedes Randintervall unterschiedlichen, Wert Yₖₒₙₛₜ.

Nach Abschluss des Erstellung des erneuerten Parametersatzes, wodurch der zweite Analyseschritt 25 beendet ist, wird der erneuerte Parametersatz in dem durch den Pfeil 27 gekennzeichneten Übergabeschritt als Hauptparametersatz an den Steuersignalpfad 3 zur Verwendung im Schritt 5 übergeben. Somit wird der erneuerte Parametersatz dann bei der weiteren Bestimmung des Steuersignals H für die Steuerung des Beatmungsgeräts 7 verwendet.

Da die Analyseschritte 21, 25 sowie der Verzögerungsschritt 23 kontinuierlich parallel zu dem Steuersignalpfad 3 ausgeführt werden, wird bei dem hierin beschriebenen Verfahren der im Steuersignalpfad 3 verwendete Hauptparametersatz ebenfalls kontinuierlich angepasst. Das bedeutet, dass die Ermittlung des Parametersatzes im Nebensegmentierungspfad kontinuierlich erfolgt, der dabei neu bestimmte Hauptparametersatz aber bevorzugt nur zu bestimmten Zeitpunkten an den Hauptsegmentierungspfad übergeben wird.

Die im Analysepfad 19 ermittelte Bewertungsfunktion bzw. der Parametersatz erlaubt, für das Verfahren wichtige Indizes zu bestimmen.

Als grundsätzliche Form der Bewertungsfunktion bzw. der in der Tabelle enthaltenen Bewertungssignalwerte wird eine nach oben geöffnete Funktion erwartet, die für negative Werte monoton fällt und für positive Werte monoton steigt. Außerdem wird für betragsmäßig kleine Werte ein negatives und für betragsmäßig große Werte ein positives Ergebnis erwartet.

Sollte die Bewertungsfunktion keine Nullstellen aufweisen, bedeutet dies, dass nicht zwischen den zwei Zuständen entschieden werden kann, und deutet auf fehlerhafte Signale hin. Der mögliche Fall, dass es mehr als zwei Nullstellen gibt, wäre ein Indiz von Artefakten und bei annähernd normalverteilten Signalen nicht zu erwarten.

In Bezug auf die Steigung der Bewertungsfunktion in Abhängigkeit von den möglichen Signalwerten gilt, dass eine monoton wachsende Steigung bei normalen Signalen zu erwarten ist. Ein besonders hoher Steigungsbetrag (z.B. an den Nullstellen) deutet auf eine sehr scharfe Trennung der beiden Zustände hin und ein besonders kleiner Steigungsbetrag an den Nullstellen auf eine sehr unscharfe Trennung.

Bei den zu erwartenden artefaktfreien Signalen ist nur der Betrag des Bewertungssignals F entscheidend. Von daher ist eine Symmetrie des Bewertungssignal in Bezug auf die Y-Achse zu erwarten. Eine Asymmetrie hingegen deutet wieder auf Artefakte hin, beispielsweise auf nicht hinreichend entfernte QRS-Komplexe des EKG-Signals.

Der horizontale Abstand vom Ursprung des Scheitelpunkts der Bewertungsfunktion ist erwartungsgemäß für Eingangswerte gleich Null, da von mittelwertfreien Signalen ausgegangen wird. Die vertikale Verschiebung muss negativ sein, da es sonst keine Nullstellen gibt.

Schließlich kann die Abweichung zwischen der geschätzten Bewertungsfunktion und einer vereinfachten quadratischen Bewertungsfunktion bestimmt werden, wobei bei einem artefaktfreien Signalen eine Ähnlichkeit zur quadratischen AMF erwartet. Eine Abweichung vom quadratischen Verlauf bedeutet, dass die Verteilungen von der Normalverteilung abweichen.

Auf der Grundlage von Abweichungen der beschriebenen zu erwartenden Merkmalen der Bewertungsfunktion können Qualitätsindizes definiert werden. Mittels der Qualitätsindizes kann das Verfahren auf Plausibilität überprüft werden und bei negativem Ergebnis beispielsweise ein Neustart des Verfahrens erfolgen. So kann u.a. dann ein Neustart erfolgen, wenn die Bewertungsfunktion zu flach ist oder sogar gegenüber der Abszisse gegenüber der erwarteten Form gespiegelt ist.

Aus dem Vorstehenden ergibt sich im Übrigen, dass das Ausführungsbeispiel der erfindungsgemäßen Vorrichtung 2 zum Erzeugen des Steuersignals H neben dem Signaleingang 4 in einem Steuersignalpfad 3 vorgesehenen Mittel zum Transformieren des Elektromyogramm-Signals E in ein geglättetes Bewertungssignal G und Mittel und zum Generieren des Steuersignals H selbst aufweist. Im Übrigen sind die Mittel zum Transformieren derart ausgestaltet, dass der Hauptparametersatz als Tabelle in der Vorrichtung hinterlegt werden kann. Außerdem sind in der Vorrichtung Mittel zum Transformieren des Analyseabschnitts E' des Elektromyogramm-Signals E in das zweite Bewertungssignal F' durch Anwenden der Bewertungsfunktion auf den Analyseabschnitt E' sowie Mittel zum Bestimmen der ersten und zweiten Häufigkeitsverteilung H₁(X), H₂(X), Mittel zum Bestimmen des erneuerten Parametersatzes und Mittel zum Übergeben des erneuerten Parametersatzes vorgesehen, wobei die Mittel zum Transformieren des Analyseabschnitts E', die Mittel zum Bestimmen der ersten und zweiten Häufigkeitsverteilung H₁(X), H₂(X), die Mittel zum Bestimmen des erneuerten Parametersatzes und die Mittel zum Übergeben des erneuerten Parametersatzes ausgestaltet sind, parallel zu den Mittel zum Transformieren des Elektromyogramm-Signals E und denen zum Generieren des Steuersignals H zu arbeiten. Außerdem sind in der Vorrichtung 2 Mittel vorgesehen, mit denen das zuvor beschriebene Analysesignal A erzeugt wird.

Bisher ist das hierin beschriebene Verfahren bzw. die erfindungsgemäße Vorrichtung 2 im Wesentlichen anhand der Fig. 1 und 2 beschrieben worden. Fig. 3 zeigt den Ablauf noch einmal anhand der dabei generierten Signale, wobei die Signale zeitlich parallel übereinander dargestellt sind, sodass erkennbar wird, dass der Ablauf im Steuersignalpfad 3 gegenüber dem Analysepfad 19 beschleunigt ist.

Im Einzelnen ist auch Fig. 3 noch einmal zu entnehmen, dass im Hauptsegmentierungs- oder Steuersignalpfad 3, der hier durch einen Kasten dargestellt ist, in dem die in den Schritten 9, 11, und 13 aus dem Elektromyogramm-Signal E erzeugten Signale F, G und H zeitlich parallel angeordnet sind. Man erkennt, dass sich das Maximum im geglätteten Bewertungssignal G gegenüber den entsprechenden Ausschlägen im Bewertungssignal F aufgrund des Glättungsschrittes 11 zeitlich leicht um den Betrag ΔT verschiebt. Daraus resultiert dann, dass der Übergang des Steuersignals H von dem ersten Steuerwert 15 zum zweiten Steuerwert 17 zeitlich gegenüber den Ausschlägen im Bewertungssignal G bzw. dem Elektromyogramm-Signal E ebenfalls leicht verzögert ist. Diese Verzögerung ist aber nicht von großem Nachteil, da die Ausschläge im Elektromyogramm-Signal im Vergleich zu anderen atmungsrelevanten physiologischen Signalen so frühzeitig auftreten, dass die Umschaltung des Beatmungsgeräts auf jeden Fall für den Patienten rechtzeitig erfolgt. Außerdem wird durch die Glättung die Qualität der Bestimmung des Übergangs von Exspirations- zu Inspirationsphase signifikant verbessert.

Des Weiteren ist aus Fig. 3 zu erkennen, dass der Ablauf im Analysepfad 19 gegenüber dem Steuersignalpfad 3 verzögert ist, da beim ersten Analyseschritt 21 beim Glätten des zweiten Bewertungssignals F' und dem Erstellen des geglätteten zweiten Bewertungssignals G' über eine größere zweite Anzahl von Signalwerten Y' des zweiten Bewertungssignals F' gemittelt wird. Die damit erzielte höhere Qualität bei der Bestimmung der Häufigkeitsverteilungen H₁(x), H₂(x) im Schritt 21 führt aber nicht zu einer Verlangsamung bei der Erzeugung des Steuersignals H, da Letzteres im Steuersignalpfad 3 erfolgt.

Außerdem ist zu erkennen, dass der Analyseabschnitt E' im Verzögerungsschritt 23 zeitlich so verzögert wird, dass das verzögerte Signal I des Analyseabschnitts E' so parallel dem zweiten Analyseschritt 25 zugeführt wird, dass ein Signalwert X des Analyseabschnitts E' zeitgleich zum dem Signalwert des Analysesignals A bearbeitet wird, der für diesen Signalwert X im ersten Analyseschritt 21 erzeugt worden ist. Das Analysesignal A stellt somit einen Indikator für die Zugehörigkeit der Signalwerte X des Analyseschnitts E' zu dem ersten oder dem zweiten Zustand bzw. die Inspirationsphase oder die Exspirationsphase dar.

In Fig. 4 ist in schematischer Form der Aufbau eines Ausführungsbeispiels einer erfindungsgemäßen Vorrichtung 2 zum Erzeugen eines Steuersignals H für ein Beatmungsgerät 7 gemäß dem zuvor beschriebenen Verfahren dargestellt. Die Vorrichtung weist einen Signaleingang 4 auf, mit dem von einem Vorverstärker 29 verstärkte Elektromyogramm-Signale E erfasst werden, die mit Hilfe von Elektroden 31 abgenommen wurden. Die Vorrichtung 2 umfasst zunächst einen Analog-Digital-Wandler 33 zum Digitalisieren des Elektromyogramm-Signals E. Dem Wandler 33 ist ein Signalprozessor 35 nachgeschaltet, mit dem das eingehenden Elektromygramm-Signal E vorverarbeitet wird, indem beispielsweise ein niederfrequenter Untergrund abgezogen wird und Signalanteile entfernt werden, die offensichtliche Artefakte sind.

Dem Signalprozessor 35 wiederum ist der eigentliche Prozessor 37 nachgeschaltet, der auf einen Speicher 39 zugreift, in dem in einem Programmspeicher 41 das Programm zu Verarbeiten der eintreffenden Daten hinterlegt ist und in dem in einem Datenspeicher 43 die Parametersätze gespeichert werden. Somit werden durch den Prozessor 37 zusammen mit dem Speicher 39 die Mittel zum Transformieren des Elektromyogramm-Signals E in ein Bewertungssignal F durch Anwenden einer Bewertungsfunktion auf das Elektromyogramm-Signal E bereitgestellt, wobei beim Transformieren einem Signalwert x des Elektromyogramm-Signals E ein im Datenspeicher 43 hinterlegter Signalwert y des Bewertungssignals F zugewiesen wird. Dieser Signalwert y wird durch den im Datenspeicher 43 als Tabelle hinterlegten Hauptparametersatz bestimmt. Außerdem stellt der Prozessor 37 auch das Mittel dar, mit dem das Steuersignal H generiert wird, mit dem das Beatmungsgerät 7 in eine Inspirationsbetriebsart oder eine Exspirationsbetriebsart geschaltet wird.

Schließlich werden durch den Prozessor 37 zusammen mit dem Datenspeicher 43 werden auch die Mittel zum bereits beschriebenen Transformieren eines Analyseabschnitts E' des Elektromyogramm-Signals E in ein zweites Bewertungssignal F' durch Anwenden der Bewertungsfunktion auf den Analyseabschnitt E' realisiert, wobei die Bewertungsfunktion durch einen im Datenspeicher 43 hinterlegten Analyseparametersatz bestimmt ist. Durch Prozessor 37 und Datenspeicher 43 werden in der schon beschriebenen Weise die erste Häufigkeitsverteilung H₁(X) und die zweite Häufigkeitsverteilung H₂(X) für die Signalwerte X des Bewertungssignals F' bestimmt und daraus ein erneuerter Parametersatz, der als Hauptparametersatz übergeben wird. Dies erfolgt durch den Prozessor 37 parallel zum Transformieren des Elektromyogramm-Signals E und dem Generieren des Steuersignals H.

Insgesamt ermöglichen das zuvor beschriebene Verfahren sowie die erfindungsgemäß ausgestaltete Vorrichtung 2, in zuverlässiger Weise aus einem Elektromyogramm-Signal E ein Steuersignal H für ein Beatmungsgerät 7 zu erzeugen, wobei dies mit einer hinreichend schnellen Verarbeitungsgeschwindigkeit erfolgt, ohne dass dies auf Kosten der Genauigkeit geht.

Außerdem passen sich das Verfahren bzw. die in dem Datenspeicher hinterlegten Parameter adaptiv selbst an Änderungen der Bedingungen an, da der zur Zuordnung der Elektromyogramm-Signalabschnitte zur Inspirationsphase bzw. Exspirationsphase erforderliche Parametersatz kontinuierlich neu bestimmt wird.

### Bezugszeichenliste:

- 1: Erfassungsschritt
- 2: Steuervorrichtung
- 3: Steuersignalpfad
- 4: Signaleingang
- 5: Schritt zum Erzeugen eines Steuersignals
- 7: Beatmungsgerät
- 9: Transformationsschritt
- 11: Glättungsschritt
- 13: Schwellwertvergleichsschritt
- 15: erster Steuerwert
- 17: zweiter Steuerwert
- 19: Analysepfad
- 21: erster Analyseschritt
- 23: Verzögerungsschritt
- 25: zweiter Analyseschritt
- 27: Übergabeschritt
- 29: Vorverstärker
- 31: Elektroden
- 33: Anlag-Digital-Wandler
- 35: Signalprozessor
- 37: Prozessor
- 39: Speicher
- 41: Programmspeicher
- 43: Datenspeicher

- E: Elektromyogramm-Signal
- E': Analyseabschnitt
- F: Bewertungssignal
- F': zweites Bewertungssignal
- G: geglättetes Bewertungssignal
- G': geglättetes zweites Bewertungssignal
- H: Steuersignal
- A: Analysesignal
- I: verzögertes Signal des Analyseabschnitts
- x: Signalwert Elektromyogramm-Signal
- X: Signalwert Analyseabschnitt
- y: Signalwert Bewertungssignal
- y': vorgegebener Signalwert Bewertungssignal
- Y: Signalwert zweites Bewertungssignal
- Y': vorgegebener Signalwert zweites Bewertungssignal

## Patentansprüche

1. Vorrichtung zum Erzeugen eines Steuersignals (H) für Beatmungsgerät (7), die einen Signaleingang zum Erfassen eines Elektromyogramm-Signals (E), das zeitlich aufeinanderfolgende Signalwerte (x) umfasst, aufweist sowie
- Mittel zum Transformieren des Elektromyogramm-Signals (E) in ein Bewertungssignal (F, G) durch Anwenden einer Bewertungsfunktion auf das Elektromyogramm-Signal (E), wobei beim Transformieren einem Signalwert (x) des Elektromyogramm-Signals (E) ein Signalwert (y) des Bewertungssignals (F, G) zugewiesen wird,
wobei die Bewertungsfunktion durch einen Hauptparametersatz bestimmt ist, sodass der Hauptparametersatz festlegt, welchem Signalwert (x) des Elektromyogramm-Signals (E) beim Transformieren bei Anwendung der Bewertungsfunktion welcher Signalwert (y) des Bewertungssignals (F, G) zugewiesen wird, und
wobei die Höhe des Signalwerts (y) des Bewertungssignals (F, G) angibt, ob das Elektromyogramm-Signal (E) einer Inspirationsphase oder einer Exspirationsphase zuzuordnen ist, wobei bei Anwendung der Bewertungsfunktion auf Signalwerte (x) aus einem Intervall um einen Basiswert ein Signalwert (y) des Bewertungssignals (F, G) zugewiesen wird und die Bewertungsfunktion der Logarithmus des Verhältnisses der Wahrscheinlichkeiten P_{I}(x), P_{E}(x) ist, dass der Signalwert (x) des Elektromyogramm-Signals (E) zu der Inspirationsphase oder zu der Exspirationsphase gehört,
- Mittel zum Generieren eines Steuersignals (H) aus zeitlich aufeinander folgenden Signalwerten, das angepasst ist, ein Beatmungsgerät (7) in eine Inspirationsbetriebsart zu schalten, wenn das Bewertungssignal (F, G) der Inspirationsphase entspricht, und in eine Exspirationsbetriebsart zu schalten, wenn das Bewertungssignal (F, G) der Exspirationsphase entspricht, und
- Mittel, die, parallel zum Transformieren des Elektromyogramm-Signals (E) und dem Generieren des Steuersignals (H), zum Analysieren zumindest eines Teils des Elektromyogramm-Signals als dessen Analyseabschnitt mit folgenden Mitteln zur Anpassung des Hauptparametersatzes eingerichtet sind:
- Mitteln zum Transformieren eines Analyseabschnitts (E') des Elektromyogramm-Signals (E) in ein zweites Bewertungssignal (F', G') durch Anwenden der Bewertungsfunktion auf den Analyseabschnitt (E'), wobei beim Transformieren einem Signalwert (X) des Analyseabschnitts (E') ein Signalwert (Y) des zweiten Bewertungssignals (F', G') zugewiesen wird,
wobei die Bewertungsfunktion durch einen Analyseparametersatz bestimmt ist, sodass der Analyseparametersatz festlegt, welchem Signalwert (X) des Analyseabschnitts (E') beim Transformieren bei Anwendung der Bewertungsfunktion welcher Signalwert (Y) des zweiten Bewertungssignals (F', G') zugewiesen wird, und
wobei die Höhe des Signalwerts (Y') des zweiten Bewertungssignals (F', G') angibt, ob der korrespondierende Signalwert (X) des Analyseabschnitts (E') einer Inspirationsphase oder einer Exspirationsphase entspricht,
- Mitteln zum Bestimmen einer ersten Häufigkeitsverteilung H₁(X) für die Signalwerte (X) aus den Teilen des Analyseabschnitts (E'), für die das zweite Bewertungssignal (F', G') angibt, dass die Inspirationsphase vorliegt,
- Mitteln zum Bestimmen einer zweiten Häufigkeitsverteilung H₂(X) für die Signalwerte (X) aus den Teilen des Analyseabschnitts (E'), für die das zweite Bewertungssignal (F', G') angibt, dass die Exspirationsphase vorliegt,
- Mitteln zum Bestimmen eines erneuerten Parametersatzes aus den Häufigkeitsverteilungen H₁(X), H₂(X) und
- Mitteln zum Übergeben des erneuerten Parametersatzes als Hauptparametersatz, wobei die Mittel zum Bestimmen der ersten und zweiten Häufigkeitsverteilung H₁(X), H₂(X), die Mittel zum Bestimmen des erneuerten Parametersatzes und die Mittel zum Übergeben des erneuerten Parametersatzes ausgestaltet sind, parallel zu den Mittel zum Transformieren des Elektromyogramm-Signals (E) und denen zum Generieren des Steuersignals (H) zu arbeiten.

2. Vorrichtung nach Anspruch 1, wobei die Mittel zum Transformieren derart ausgestaltet sind, dass der Hauptparametersatz als Tabelle hinterlegt ist, in der für jeden möglichen Signalwert (x) des Elektromyogramm-Signals (E) der zugewiesene Signalwert (y) für das Bewertungssignal (F, G) enthalten ist, wobei bei der Anwendung der Bewertungsfunktion einem Signalwert (x) des Elektromyogramm-Signals (E) der der Tabelle entsprechende Wert als Signalwert (y) des Bewertungssignals Z (F, G) zugewiesen wird.

3. Vorrichtung nach einem der Ansprüche 1 bis 2, wobei Mittel vorgesehen sind, ein Analysesignal (A) zu generieren, das für jeden Signalwert (X) des Analyseabschnitts (E') einen Analysesignalwert enthält,
wobei ein Analysesignalwert einen ersten Wert annimmt, wenn derjenige Signalwert des zweiten Bewertungssignals (F', G'), der dem Signalwert (X) des Analyseabschnitts (E') entspricht, zu dem der Analysesignalwert generiert wird, oberhalb eines Schwellwerts liegt,
wobei ein Analysesignalwert einen zweiten Wert annimmt, wenn derjenige Signalwert (X) des zweiten Bewertungssignals (F', G'), der dem Signalwert des Analyseabschnitts (E') entspricht, zu dem der Analysesignalwert generiert wird, unterhalb eines Schwellwerts liegt,
und wobei die Mittel zum Bestimmen der Häufigkeitsverteilungen H₁(X), H₂(X) derart angepasst sind, dass die erste Häufigkeitsverteilung H₁(X) für die Signalwerte (X) des Analyseabschnitts (E') bestimmt wird, für die der Analysesignalwert den ersten Wert hat, die zweite Häufigkeitsverteilung H₂(X) für die Signalwerte (X) des Analyseabschnitts (E') bestimmt wird, für die der Analysesignalwert den zweiten Wert hat.

## Claims

1. A device for generating a control signal (H) for a respiratory apparatus (7) that has a signal input for detecting an electromyogram signal (E) which comprises signal values (x) following each other in time, and also
- means for transforming the electromyogram signal (E) into an evaluation signal (F, G) by applying an evaluation function to the electromyogram signal (E), wherein during the transformation a signal value (y) of the evaluation signal (F, G) is assigned to a signal value (x) of the electromyogram signal (E),
wherein the evaluation function is determined by a main parameter set so that the main parameter set establishes which signal value (y) of the evaluation signal (F, G) is assigned to which signal value (x) of the electromyogram signal (E) during the transformation when applying the evaluation function, and
wherein the height of the signal value (y) of the evaluation signal (F, G) indicates whether the electromyogram signal (E) is to be associated with an inspiration phase or an expiration phase, wherein a signal value (y) of the evaluation signal (F, G) is assigned, when applying the evaluation function, to signal values (x) from a range around a base value, and the evaluation function is the logarithm of the ratio of the probabilities P_{I}(x), P_{E}(x) that the signal value (x) of the electromyogram signal (E) belongs to the inspiration phase or to the expiration phase,
- means for generating a control signal (H) from signal values following each other in time that is adapted to switch a respiratory apparatus (7) into an inspiration operating mode when the evaluation signal (F, G) corresponds to the inspiration phase and to switch it into an expiration operating mode when the evaluation signal (F, G) corresponds to the expiration phase, and
- means which are set up to analyse, parallel to the transformation of the electromyogram signal (E) and the generation of the control signal (H), at least a portion of the electromyogram signal as the analysis segment thereof with the following means for adaptation of the main parameter set:
- means for transforming an analysis segment (E') of the electromyogram signal (E) into a second evaluation signal (F', G') by applying the evaluation function to the analysis segment (E'), wherein during the transformation a signal value (Y) of the second evaluation signal (F', G') is assigned to a signal value (X) of the analysis segment (E'),
wherein the evaluation function is determined by an analysis parameter set so that the analysis parameter set establishes which signal value (Y) of the second evaluation signal (F', G') is assigned to which signal value (X) of the analysis segment (E') during the transformation when applying the evaluation function, and
wherein the height of the signal value (Y') of the second evaluation signal (F', G') indicates whether the corresponding signal value (X) of the analysis segment (E') corresponds to an inspiration phase or an expiration phase,
- means for determining a first frequency distribution H₁(X) for the signal values (X) from the portions of the analysis segment (E') for which the second evaluation signal (F', G') indicates that the inspiration phase is present,
- means for determining a second frequency distribution H₂(X) for the signal values (X) from the portions of the analysis segment (E') for which the second evaluation signal (F', G') indicates that the expiration phase is present,
- means for determining a renewed parameter set from the frequency distributions H₁(X), H₂(X), and
- means for transferring the renewed parameter set as a main parameter set,
wherein the means for determining the first and second frequency distribution H₁(X), H₂(X), the means for determining the renewed parameter set and the means for transferring the renewed parameter set are configured to operate parallel to the means for transforming the electromyogram signal (E) and those for generating the control signal (H).

2. A device according to claim 1, wherein the means for the transformation are configured in such a way that the main parameter set is stored as a table in which the assigned signal value (y) for the evaluation signal (F, G) is contained for each possible signal value (x) of the electromyogram signal (E), wherein during the application of the evaluation function the value corresponding to the table is assigned as a signal value (y) of the evaluation signal (F, G) to a signal value (x) of the electromyogram signal (E).

3. A device according to one of claims 1 to 2, wherein means are provided to generate an analysis signal (A) which contains an analysis signal value for each signal value (X) of the analysis segment (E'),
wherein an analysis signal value assumes a first value when that signal value of the second evaluation signal (F', G') which corresponds to the signal value (X) of the analysis segment (E'), for which the analysis signal value is generated, lies above a threshold value,
wherein an analysis signal value assumes a second value when that signal value (X) of the second evaluation signal (F', G') which corresponds to the signal value of the analysis segment (E'), for which the analysis signal value is generated, lies below a threshold value,
and wherein the means for determining the frequency distributions H₁(X), H₂(X) are adapted in such a way that the first frequency distribution H₁(X) is determined for the signal values (X) of the analysis segment (E') for which the analysis signal value has the first value; the second frequency distribution H₂(X) is determined for the signal values (X) of the analysis segment (E') for which the analysis signal value has the second value.

## Revendications

1. Dispositif pour générer un signal de commande (H) pour un respirateur (7), qui présente une entrée de signal pour détecter un signal d'électromyogramme (E) qui comprend des valeurs de signal (x) successives dans le temps et
- des moyens pour transformer le signal d'électromyogramme (E) en un signal d'évaluation (F, G) par application d'une fonction d'évaluation sur le signal d'électromyogramme (E), une valeur de signal (y) du signal d'évaluation (F, G) étant assignée à une valeur de signal (x) du signal d'électromyogramme (E) lors de la transformation,
la fonction d'évaluation étant déterminée par un jeu de paramètres principal de telle sorte que le jeu de paramètres principal définit à quelle valeur de signal (x) du signal d'électromyogramme (E) est assignée quelle valeur de signal (y) du signal d'évaluation (F, G) lors de la transformation lorsque la fonction d'évaluation est appliquée, et
le niveau de la valeur de signal (y) du signal d'évaluation (F, G) indiquant si le signal d'électromyogramme (E) doit être assigné à une phase d'inspiration ou à une phase d'expiration, une valeur de signal (y) du signal d'évaluation (F, G) étant assignée lorsque la fonction d'évaluation est appliquée sur des valeurs de signal (x) issues d'un intervalle autour d'une valeur de base et la fonction d'évaluation étant le logarithme du rapport des probabilités P_{I}(x), P_{E}(x) que la valeur de signal (x) de l'électromyogramme (E) appartienne à la phase d'inspiration ou à la phase d'expiration,
- des moyens pour générer un signal de commande (H) à partir de valeurs de signal successives dans le temps, qui est adapté à faire passer un respirateur (7) en mode inspiration lorsque le signal d'évaluation (F, G) correspond à la phase d'inspiration et à le faire passer en mode expiration lorsque le signal d'évaluation (F, G) correspond à la phase d'expiration ; et
- des moyens qui sont réalisés pour, en parallèle à la transformation du signal d'électromyogramme (E) et à la génération du signal de commande (H), analyser au moins une partie du signal d'électromyogramme en tant que sa section d'analyse, avec les moyens suivants pour adapter le jeu de paramètres principal :
- des moyens pour transformer une section d'analyse (E') du signal d'électromyogramme (E) en un deuxième signal d'évaluation (F', G') en appliquant la fonction d'évaluation sur la section d'analyse (E'), une valeur de signal (Y) du deuxième signal d'évaluation (F', G') étant assignée à une valeur de signal (X) de la section d'analyse (E') lors de la transformation,
la fonction d'évaluation étant déterminée par un jeu de paramètres d'analyse de telle sorte que le jeu de paramètres d'analyse définit à quelle valeur de signal (X) de la section d'analyse (E') est assignée quelle valeur de signal (Y) du deuxième signal d'évaluation (F', G') lors de la transformation lorsque la fonction d'évaluation est appliquée, et
le niveau de la valeur de signal (Y') du deuxième signal d'évaluation (F', G') indiquant si la valeur de signal (X) correspondante de la section d'analyse (E') correspond à une phase d'inspiration ou à une phase d'expiration,
- des moyens pour déterminer une première distribution de fréquences H₁(X) pour les valeurs de signal (X) issues des parties de la section d'analyse (E') pour lesquelles le deuxième signal d'évaluation (F', G') indique la présence de la phase d'inspiration,
- des moyens pour déterminer une deuxième distribution de fréquences H₂(X) pour les valeurs de signal (X) issues des parties de la section d'analyse (E') pour lesquelles le deuxième signal d'évaluation (F', G') indique la présence de la phase d'expiration,
- des moyens pour déterminer un nouveau jeu de paramètres à partir des distributions de fréquences H₁(X), H₂(X) et
- des moyens pour délivrer le nouveau jeu de paramètres en tant que jeu de paramètres principal, les moyens pour déterminer les première et deuxième distributions de fréquences H₁(X), H₂(X), les moyens pour déterminer le nouveau jeu de paramètres et les moyens pour délivrer le nouveau jeu de paramètres étant conçus pour fonctionner en parallèle avec les moyens pour transformer le signal d'électromyogramme (E) et ceux pour générer le signal de commande (H).

2. Dispositif selon la revendication 1, dans lequel les moyens de transformation sont réalisés de telle sorte que le jeu de paramètres principal est stocké sous la forme d'un tableau qui contient, pour chaque valeur de signal (x) possible du signal d'électromyogramme (E), la valeur de signal (y) assignée pour le signal d'évaluation (F, G), dans lequel, lors de l'application de la fonction d'évaluation, à une valeur de signal (x) du signal d'électromyogramme (E) est assignée, en tant que valeur de signal (y) du signal d'évaluation (F, G), la valeur correspondante du tableau.

3. Dispositif selon l'une des revendications 1 à 2, dans lequel des moyens sont prévus pour générer un signal d'analyse (A) qui contient une valeur de signal d'analyse pour chaque valeur de signal (X) de la section d'analyse (E'),
dans lequel une valeur de signal d'analyse prend une première valeur lorsque la valeur de signal du deuxième signal d'évaluation (F', G'), qui correspond à la valeur de signal (X) de la section d'analyse (E') pour laquelle la valeur de signal d'analyse est générée, est supérieure à une valeur seuil,
dans lequel une valeur de signal d'analyse prend une deuxième valeur lorsque la valeur de signal (X) du deuxième signal d'évaluation (F', G'), qui correspond à la valeur de signal de la section d'analyse (E') pour laquelle la valeur de signal d'analyse est générée, est inférieure à une valeur seuil,
et dans lequel les moyens pour déterminer les distributions de fréquences H₁(X), H₂(X) sont adaptés de telle sorte que la première distribution de fréquences H₁(X) est déterminée pour les valeurs de signal (X) de la section d'analyse (E') pour lesquelles la valeur de signal d'analyse a la première valeur, la deuxième distribution de fréquences H₂(X) est déterminée pour les valeurs de signal (X) de la section d'analyse (E') pour lesquelles la valeur de signal d'analyse a la deuxième valeur.
